(19) Europäisches Patentamt
European Patent Office
Office européen des brevets

(11) **EP 4 361 635 A1**

(12) **EUROPEAN PATENT APPLICATION**
published in accordance with Art. 153(4) EPC

(43) Date of publication:
**01.05.2024 Bulletin 2024/18**

(51) International Patent Classification (IPC):
**G01N 33/68** (2006.01)    **G01N 27/447** (2006.01)
**G06N 3/02** (2006.01)

(21) Application number: **22871369.9**

(22) Date of filing: **14.04.2022**

(86) International application number:
**PCT/CN2022/086799**

(87) International publication number:
**WO 2023/045306 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.09.2021 CN 202111114069**

(71) Applicant: **Beijing Xiaoying Technology Co., Ltd.
Beijing 100084 (CN)**

(72) Inventors:
• **LIAN, Heqing
  Beijing 100084 (CN)**
• **LI, Bairui
  Beijing 100084 (CN)**

(74) Representative: **LLR
2, rue Jean Lantier
75001 Paris (FR)**

(54) **MONOCLONAL IMMUNOGLOBULIN QUANTITATIVE ANALYSIS METHOD USING CAPILLARY IMMUNOTYPING**

(57) The present application provides a quantitative analysis method of a capillary immunotyping monoclonal immune globulin, performing electrophoresis on a monoclonal immune globulin serum sample to obtain an M protein curve, mixing the monoclonal immune globulin serum sample with an antibody to form an immunocomplex and then performing electrophoresis again to obtain an M protein post-reaction curve, compounding the M protein curve with the M protein post-reaction curve, determining a region boundary through curve derivation or a neural network, and then performing quantitative calculation to obtain a quantitative result of an M protein. In order to solve a problem of M protein type identification, the application combines a immunochemical method and an electrophoresis method by using a capillary immunotyping technology, wherein electrophoresis is performed after a specific antibody and a corresponding M protein form an immunocomplex, thus an M protein peak will be eliminated, and the M protein can be correctly quantified through a difference value between peak areas of twice electrophoresis. The method of the present application can objectively and correctly distinguish the M protein from other non-immune globulins, and avoids problems of manual quantification in terms of subjectivity and poor precision.

FIG. 5

EP 4 361 635 A1

**Description**

**CROSS REFERENCE**

The present application claims the priority of Chinese Patent Application No. 202111114069.3 filed on September 23, 2021 and entitled "QUANTITATIVE ANALYSIS METHOD OF CAPILLARY IMMUNOTYPING MONOCLONAL IMMUNE GLOBULIN", the disclosure of which is incorporated by reference herein in its entirety.

**TECHNICAL FIELD**

The present application relates to the technical field of measurement and testing, particularly to a quantitative analysis method of a capillary immunotyping monoclonal immune globulin.

**BACKGROUND**

At present, quantification of M protein (monoclonal immune globulin) is achieved by using a serum protein electrophoresis method. Its principle is that if a narrow bottomed peak appears in a serum protein electrophoresis spectrum, the protein is considered as the M protein and quantified after being selected via software.

When the M protein has low content and migrates in a $\beta$ or $\alpha$ region, its protein peak cannot be distinguished from the protein peaks of other non-immune globulins, and the objectivity of its results cannot be guaranteed after being selected or being subjectively selected, thereby affecting the diagnosis and efficacy evaluation of related diseases.

**SUMMARY**

In order to solve a problem of M protein type identification, the present application combines an immunochemical method and an electrophoresis method by adopting a capillary immunotyping technology, wherein electrophoresis is performed after a specific antibody and a corresponding M protein form an immunocomplex, thus the M protein peak will be eliminated, and the M protein can be accurately quantified by a difference value between peak areas of two electrophoresis. When the migration position of the M protein is the $\beta$ region, the M protein that often belongs to IgA class, the method of the present application is particularly suitable for objectively and accurately distinguishing the M protein from other non-immune globulins, so as to avoid problems of manual quantification in terms of the subjectivity and poor precision. The present application provides a quantitative analysis method of a capillary immunotyping monoclonal immune globulin, wherein a region boundary of a compound curve is determined through curve derivation or a neural network, and then quantitative calculation is performed to obtain a quantitative result of the M protein;

the compound curve is obtained by the following method:

performing electrophoresis on a monoclonal immune globulin serum sample to obtain an M protein curve; mixing the monoclonal immune globulin serum sample with an antibody to form an immunocomplex and then performing electrophoresis again to obtain an M protein post-reaction curve; and

compounding the M protein curve with the M protein post-reaction curve to obtain a compound curve.

The quantitative analysis method of capillary immunotyping monoclonal immune globulin of the present application, as a preferred mode, comprises the following steps:

S1, obtaining a M protein curve and an M protein post-reaction curve: performing electrophoresis on a monoclonal immune globulin serum sample to obtain an M protein curve, mixing the monoclonal immune globulin serum sample with an antibody to form an immunocomplex and then performing electrophoresis again to obtain an M protein post-reaction curve;

S2, compounding and determining a region boundary line: compounding the M protein curve with the M protein post-reaction curve to obtain a compound curve, and determining a region boundary of a compound curve through curve derivation or a neural network;

the region boundary comprises a left boundary line of a $\beta$ region, a right boundary line of the $\beta$ region, a left boundary line of a $\gamma$ region and a right boundary line of the $\gamma$ region ;

a method for determining the region boundary through curve derivation is as follows: calculating a first-order derivative and a second-order derivative of each point on the M protein curve, and determining the region boundary according to the values of the first-order derivative and the second-order derivative;

S3, quantitative calculation: calculating the area $S_{ELP}$ of the M protein curve in the $\beta$ region or the $\gamma$ region according to the left boundary line of the $\beta$ region, the right boundary line of the $\beta$ region, and/or the left boundary line of the γ region, the right boundary line of the γ region, calculating the area $S_x$ of the M protein post-reaction curve in the β region or the γ region according to the left boundary line of the β region, the right boundary line of the β region or the left boundary line of the γ region, the right boundary line of the γ region, and calculating the quantitative result V of the M protein according to the following equation:

$$V = (S_{ELP}-S_x)*K,$$

wherein, K is a coefficient.

In the quantitative analysis method of the capillary immunotyping monoclonal immune globulin of the present application, as a preferred mode, the antibody comprises IgA antibody, IgG antibody and IgM antibody, each of which comprises K type and L type;
both the M protein curve and the M protein post-reaction curve comprise 300 coordinate points.

In the quantitative analysis method of the capillary immunotyping monoclonal immune globulin of the present application, as a preferred mode, in step S2, a coordinate point position where the first-order derivative is 0 is a wave peak or wave trough of the M protein curve, the left boundary line of the β region is a first wave trough in a designated region, the right boundary line of the β region is a second wave trough or a position where a first second-order derivative is 0 after a first peak, the left boundary line of the γ region is a first wave trough after the right boundary line of the β region, the right boundary line of the γ region is a first coordinate point position where the difference between the M protein curve and the M protein post-reaction curve is 0.

In the quantitative analysis method of the capillary immunotyping monoclonal immune globulin of the present application, as a preferred mode, in step S2, the equation of the first-order derivative is

$$f'(x_0) = \lim_{\Delta x \to 0} \frac{\Delta y}{\Delta x} = \lim_{\Delta x \to 0} \frac{f(x_0 + \Delta x) - f(x_0)}{\Delta x}.$$

In the quantitative analysis method of the capillary immunotyping monoclonal immune globulin of the present application, as a preferred mode, in step S2, the equation of the second-order derivative is

$$f''(x) = \lim_{\Delta x \to 0} \frac{f'(x_0 + \Delta x) - f'(x_0)}{\Delta x}.$$

In the quantitative analysis method of the capillary immunotyping monoclonal immune globulin of the present application, as a preferred mode, in step S2, the designated region is a region where a monoclonal immune globulin often appears.

In the quantitative analysis method of the capillary immunotyping monoclonal immune globulin of the present application, as a preferred mode, in step S2, a method for determining the region boundary of the compound curve through neural network is as follows: performing scaling and residual block convolution operation on the image of the compound curve to obtain a feature map and ultimately obtain a feature vector, performing classification by utilizing the feature vector to finally obtain n classification results, and then determining whether each of the classification results is the left boundary line of the β region, the right boundary line of the β region, the left boundary line of the γ region or the right boundary line of the γ region, then obtaining the region boundary.

In the quantitative analysis method of the capillary immunotyping monoclonal immune globulin of the present application, as a preferred mode, in step S2, a method for obtaining the classification results is as follows: resizing the image of the compound curve to obtain a 224*224 image which is fed into a first residual block, performing four convolution operations to obtain a 220*220 feature map, interpolating the 224*224 image to obtain a 220*220 image, and adding the 220*220 image and the 220*220 feature map to obtain a 220*220 new feature map;

then, down-sampling twice the 220*220 new feature map to obtain a 110*110 feature map which is input a second residual block, performing four convolution operations to obtain a 106* 106 new feature map, interpolating the 110*110 feature map to obtain a 106*106 feature map, adding the 106*106 feature map and the 106*106 new feature map to obtain a 106*106 fused feature map, and down-sampling twice the 106*106 fused feature map to obtain a 53*53 feature map;

the above operations are repeated to finally obtain a 10*10 feature map, which is global average pooled to obtain a 2560-dimensional feature vector, and classification is performed by utilizing the 2560-dimensional feature vector to finally obtain n of the classification results.

In the quantitative analysis method of the capillary immunotyping monoclonal immune globulin of the present application, as a preferred mode, n is 300.

In the quantitative analysis method of the capillary immunotyping monoclonal immune globulin of the present application, as a preferred mode, in step S2, a method for obtaining the compound curve is as follows: inputting the M protein curve and the M protein post-reaction curve into a database respectively, then parsing all the data of the database to call out the M protein curve, calling out the M protein post-reaction curve from the database by being paired with the M protein curve, and then compounding the M protein curve with the M protein post-reaction curve to obtain the compound curve.

The measurement principle of the present application is as follows: electrophoresis is performed after a specific antibody and the corresponding M protein form the immunocomplex, thus the M protein peak will be eliminated, and the M protein can be correctly quantified by a difference value between peak areas of twice electrophoresis. When the migration position of the M protein is the β region, the M protein that ordinarily belongs to IgA class, the method of the present application is particularly suitable for objectively and accurately distinguishing the M protein from other non-immune globulins, so as to avoid problems of manual quantification in terms of the subjectivity and poor precision.

This software is compatible with Sebia's fully automatic capillary electrophoresis meter and can intelligently and automatically parses quantitative results. The product input is a database file in formats such as MDB, and the output is the quantitative result in g/l.

Firstly, the mdb file is parsed, and data of a single curve is obtained (curve data can also be directly imported from the system ). Two corresponding curves are paired. The boundaries of the β region and the γ region of the curve are found through a neural network or curve derivation, and then the quantitative result is calculated according to the numerical values of the two curves.

### Database parsing

Firstly, a piece of data information is parsed from the database. Then, curve information is extracted from the piece of information, and this curve consists of 300 coordinate points.

### Curve pairing

For each curve of M protein such as IgA, IgG, IgM, K, L and Ref, its corresponding ELP curve (i.e., M protein post-reaction curve) is searched in the database as a reference curve.

### Region determining

Method 1: curve derivation

A first-order derivative (slope, Equation I) and a second-order derivative (Equation II) of each point on the ELP curve are calculated. The wave peak and the wave trough on the curve are found according to a position where the first-order derivative is 0, the first wave trough in the fixed region is selected as the left boundary of the β region, the second wave trough or a position where the first second-order derivative is 0 after the first wave peak is selected as the right boundary of the β region. The right boundary of the β region is taken as the left boundary of the γ region, and a first position where the difference value between two curves is 0 is taken as the right boundary.

$$f'(x_0) = \lim_{\Delta x \to 0} \frac{\Delta y}{\Delta x} = \lim_{\Delta x \to 0} \frac{f(x_0 + \Delta x) - f(x_0)}{\Delta x}.$$   Equation I

$$f''(x) = \lim_{\Delta x \to 0} \frac{f'(x_0 + \Delta x) - f'(x_0)}{\Delta x}$$

Equation II

Method 2: neural network

Through the neural network, this problem is regarded as an image classification problem, with the input being a curve image. Firstly, an input image is resized to a size of 224 * 224 and then fed into a first residual block. After four convolution operations, a feature map with a size of 220 * 220 is obtained. The input 224 * 224 image is interpolated to obtain an image with a size 220 * 220, which is added with the firstly obtained feature value to obtain a new feature map with a size of 220 * 220. Then, the feature map is down-sampled twice to obtain a 110 * 110 feature map, which is fed into a second residual block and subjected to four convolution operations to obtain a 106 * 106 new feature map. Similarly, the 110 * 110 feature map is interpolated to obtain a 106 * 106 feature map, which is added with the feature map obtained after operation of second residual block to obtain a 106 * 106 fused feature map, and this fused feature map is down-sampled twice to obtain a feature map with a size of 53 * 53. The above operations are repeated to finally obtain a feature map with a size of 10 * 10. This feature map is global average pooled to obtain a 2560-dimensional feature vector, and classification is performed by utilizing the feature vector to finally obtain a classification result for 300 classes. The classification result for each class is whether it is the boundary point of the $\beta$ or $\gamma$ region.

**Quantitative calculation**

$$V=(S_{ELP}-S_x) * K$$

V: Quantitative result
$S_{ELP}$: the area of ELP curve in $\beta$ region or $\gamma$ region
$S_x$: the area of M protein curve in $\beta$ region or $\gamma$ region
K: coefficient

K is used for dimension conversion, i.e., unit conversion.
The present application has the following advantages:
The existing method may only quantify or only qualitatively analyze M protein. The method of present application performs quantitative calculation on the basis of a qualitative method, and can achieve both qualitative and quantitative analysis of M protein. In addition, the quantitative mode of present method has better precision and higher resolution compared with the existing quantitative method, is faster and more convenient than manual quantitative methods, and can obtain the quantitative results without manual intervention.

**BRIEF DESCRIPTION OF THE DRAWINGS**

FIG. 1 is a flowchart of a quantitative analysis method of a capillary immunotyping monoclonal immune globulin;
FIG. 2 is a flowchart of database call and analysis of a quantitative analysis method of a capillary immunotyping monoclonal immune globulin;
FIG. 3 is a flowchart of determining a region boundary of a compound curve through a neural network in a quantitative analysis method of a capillary immunotyping monoclonal immune globulin;
FIG. 4 is a diagram showing M protein cannot be quantified after serum protein electrophoresis;
FIG. 5 is a diagram of a compound curve of embodiment 2 in a quantitative analysis method of a capillary immunotyping monoclonal immune globulin.

**DETAILED DESCRIPTION**

The technical solution in embodiments of the present application will be clearly and completely described in combination with drawings in embodiments of the present application. Obviously, the described embodiments are only some embodiments of the present application, but not all the embodiments.

Embodiment 1

A quantitative analysis method of a capillary immunotyping monoclonal immune globulin is as follows: a region boundary of a compound curve is determined through curve derivation or a neural network, and then quantitative calculation is performed to obtain a quantitative result of an M protein;

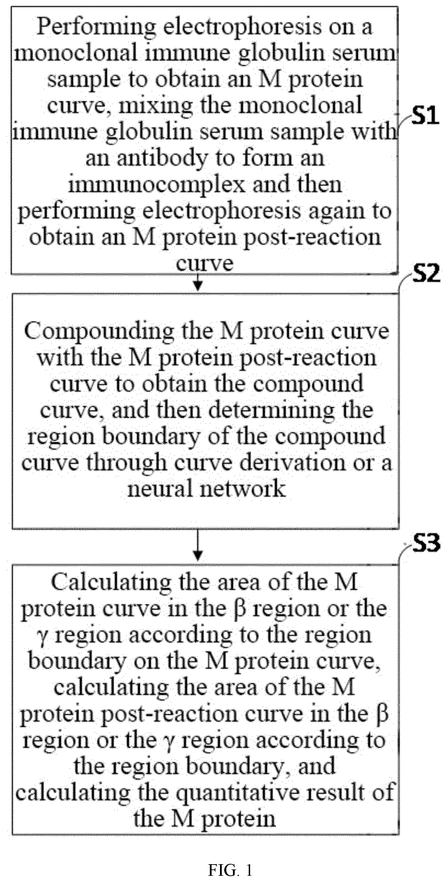

the compound curve is obtained by the following method: performing electrophoresis on a monoclonal immune globulin serum sample to obtain an M protein curve, mixing the monoclonal immune globulin serum sample with an antibody to form an immunocomplex and then performing electrophoresis again to obtain an M protein post-reaction curve, and compounding the M protein curve with the M protein post-reaction curve to obtain a compound curve; as shown in FIG. 1, the quantitative analysis method comprises the following steps:

S1, obtaining a M protein curve and an M protein post-reaction curve: performing electrophoresis on a monoclonal immune globulin serum sample to obtain an M protein curve, mixing the monoclonal immune globulin serum sample with an antibody to form an immunocomplex and then performing electrophoresis again to obtain an M protein post-reaction curve;

the antibody is IgA antibody, IgG antibody and IgM antibody, each of which comprises K type and L type; both the M protein curve and the M protein post-reaction curve comprise 300 coordinate points.

S2, compounding and determining a region boundary line: compounding the M protein curve and the M protein post-reaction curve to obtain a compound curve, and determining the region boundary of the compound curve through curve derivation or a neural network;

the region boundary comprises a left boundary line of the β region, a right boundary line of the β region, a left boundary line of the γ region and a right boundary line of the γ region;

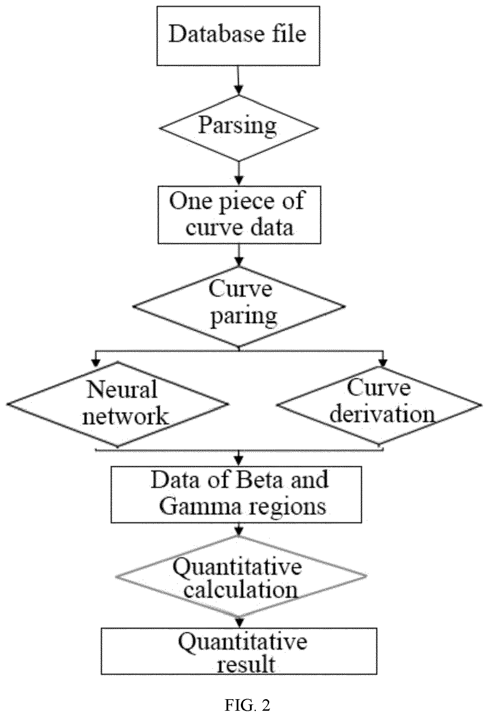

as shown in FIG. 2, a method for determining the region boundary of the compound curve through curve derivation is as follows: calculating a first-order derivative and a second-order derivative of each point on the M protein curve, and determining the region boundary according to the values of the first-order derivative and the second-order derivative;

a coordinate point position where the first-order derivative is 0 is a wave peak or wave trough of the M protein curve, the left boundary line of the β region is a first wave trough in a designated region, the right boundary line of the β region is a second wave trough or a position where a first second-order derivative is 0 after a first peak, the left boundary line of the γ region is a first wave trough after the right boundary of the β region, the right boundary line of the γ region is a first coordinate point position where the difference between the M protein curve and the M protein post-reaction curve is 0;

the equation of the first-order derivative is:

$$f'(x_0) = \lim_{\Delta x \to 0} \frac{\Delta y}{\Delta x} = \lim_{\Delta x \to 0} \frac{f(x_0 + \Delta x) - f(x_0)}{\Delta x} \quad ;$$

the equation of the second-order derivative is:

$$f''(x) = \lim_{\Delta x \to 0} \frac{f'(x_0 + \Delta x) - f'(x_0)}{\Delta x}$$

;

the designated region was a region where a monoclonal immune globulin often appear;

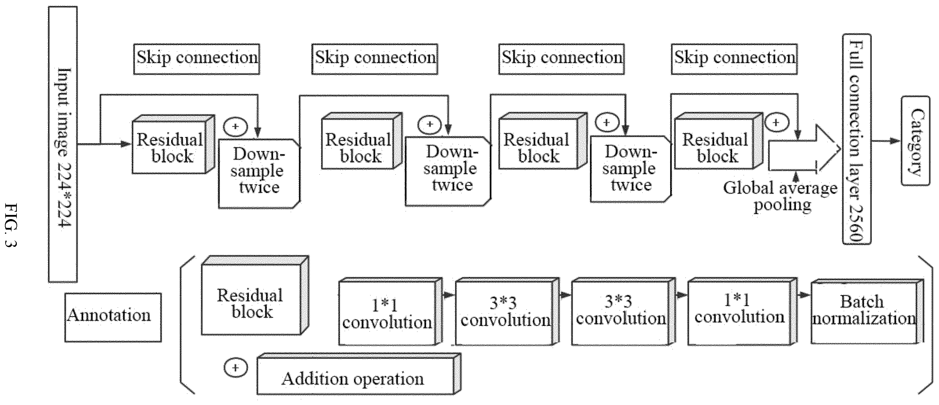

as shown in FIG. 3, a method for determining the region boundary of the compound curve through neural network is as follows: performing scaling and residual block convolution operation on the image of the compound curve to obtain a feature map and ultimately obtain a feature vector, performing classification by utilizing the feature vector to finally obtain n classification results, and then determining whether each of the classification results is the left boundary line of the β region, the right boundary line of the β region, the left boundary line of the γ region or the right boundary line of the γ region, then obtaining the region boundary;

resizing the image of the compound curve to obtain a 224*224 image which is fed into a first residual block, performing four convolution operations to obtain a 220*220 feature map, interpolating the 224*224 image to obtain a 220*220 image, and adding the 220*220 image and the 220*220 feature map to obtain a 220*220 new feature map;

then, down-sampling twice the 220*220 new feature map to obtain a 110*110 feature map which is fed into a second residual block, performing four convolution operations to obtain a 106*106 new feature map, interpolating the 110*110 feature map to obtain a 106*106 feature map, adding the 106*106 feature map and the 106*106 new feature map to obtain a 106*106 fused feature map, and down-sampling twice the 106*106 fused feature map to obtain a 53*53 feature map;

the above operations are repeated to finally obtain a 10* 10 feature map, which is global average pooled to obtain a 2560-dimensional feature vector, and classification was performed by utilizing the 2560-dimensional feature vector to finally obtain n of the classification results, wherein n is 300;

the M protein curve and the M protein post-reaction curve are compounded through the following ways: inputting the M protein curve and the M protein post-reaction curve into a database respectively, then parsing all the data of the database to call out the M protein curve, calling out the M protein post-reaction curve from the database by being paired with the M protein curve, and then compounding the M protein curve with the M protein post-reaction curve to obtain the compound curve;

S3, quantitative calculation: calculating the area $S_{ELP}$ of the M protein curve in the β region or the γ region according to the left boundary line of the β region, the right boundary line of the β region and/or the left boundary line of the γ region, the right boundary line of the γ region, calculating the area $S_x$ of the M protein post-reaction curve in the β region or the γ region according to the left boundary line of the β region, the right boundary line of the β region or the left boundary line of the γ region, the right boundary line of the γ region, and calculating the quantitative result V of the M protein according to the following equation:

$V = (S_{ELP} - S_x)*K$,

wherein, K is a coefficient.

Embodiment 2

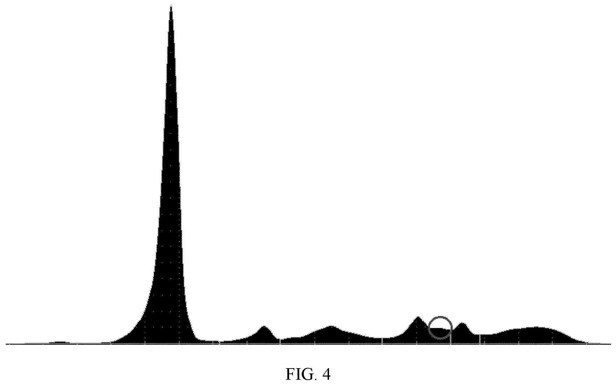

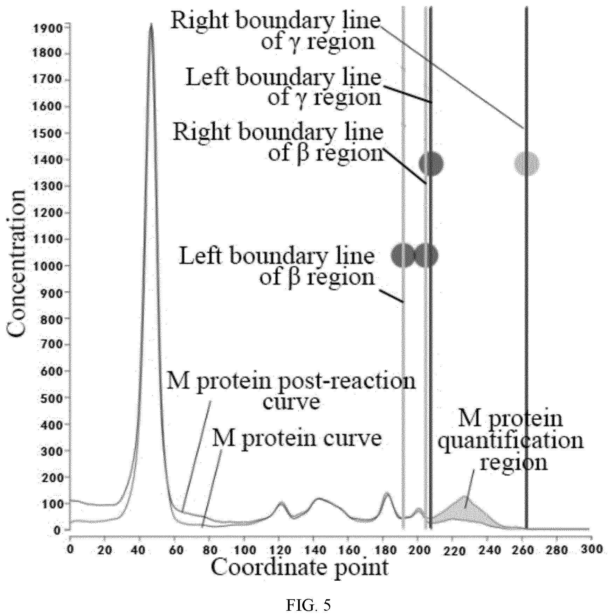

A quantitative analysis method of a capillary immunotyping monoclonal immune globulin is as follows: electrophoresis was performed after a specific antibody and a corresponding M protein formed an immunocomplex, thus the M protein peak would be eliminated, and the M protein could be correctly quantified by a difference value between peak areas of twice electrophoresis (see FIG. 4 and FIG. 5). When the migration position of the M protein is the β region, the M protein that ordinarily belonged to IgA class, the method is particularly suitable for objectively and accurately distinguishing the M protein from other non-immune globulins, so as to avoid problems of manual quantification in terms of the subjectivity and poor precision.

This software is compatible with Sebia's fully automatic capillary electrophoresis meter and can intelligently and automatically parses quantitative results.The product input is a database file in formats such as MDB, and the output is the quantitative result in g/l.

As shown in FIG. 2, firstly, the mdb file is parsed, and data of a single curve is obtained (curve data can also be directly imported from the system). Two corresponding curves are paired. The boundaries of the β region and the γ region of the curve are found through neural network or curve derivation, and then the quantitative result is calculated according to the numerical values of the two curves.

**Database parsing**

Firstly, a piece of data information is parsed from the database. Then, curve information is extracted from the piece of information, and the curve is comprised of 300 coordinate points.

**Curve pairing**

For each curve of M protein such as IgA, IgG, IgM, K, L and Ref, its corresponding ELP curve is searched in the database as a reference curve.

**Region determining**

Method 1: curve derivation

A first-order derivative (slope, Equation I) and a second-order derivative (Equation II) of each point on the ELP curve are calculated. The wave peak and the wave trough on the curve are found according to a position where the first-order derivative is 0, the first wave trough in the fixed region is selected as the left boundary of the β region, the second wave trough or a position where the first second-order derivative is 0 after the first wave peak is selected as the right boundary of the β region. The right boundary of the β region is taken as the left boundary of the γ region, and a first position where the difference value between two curves was 0 is taken as the right boundary.

$$f'(x_0) = \lim_{\Delta x \to 0} \frac{\Delta y}{\Delta x} = \lim_{\Delta x \to 0} \frac{f(x_0 + \Delta x) - f(x_0)}{\Delta x}.$$
Equation I

$$f''(x) = \lim_{\Delta x \to 0} \frac{f'(x_0 + \Delta x) - f'(x_0)}{\Delta x}$$
Equation II

Method 2: neural network

As shown in FIG. 3, through the neural network, this problem is regarded as an image classification problem, with the input being a curve image. Firstly, an input image is resized to a size of 224 * 224 and then fed into a first residual block. After four convolution operations, a feature map with a size of 220 * 220 is obtained. The input 224 * 224 image is interpolated to obtain an image with a size 220 * 220 which is added with the firstly obtained feature value to obtain a new feature map with a size of 220 * 220. Then, the feature map is down-sampled twice to obtain a 110 * 110 feature map, which is fed into a second residual block and subjected to four convolution operations to obtain a 106 * 106 new feature map. Similarly, the 110 * 110 feature map is interpolated to obtain a 106 * 106 feature map, which is added with the feature map obtained after operation of second residual block to obtain a 106 * 106 fused feature map, and this fused feature map is down-sampled twice to obtain a feature map with a size of 53 * 53. The above operations are repeated to finally obtain a feature map with a size of 10 * 10. This feature map is global average pooled to obtain a 2560-dimensional feature vector, and classification is performed by utilizing the feature vector to finally obtain a classification result for 300 classes. The classification result for each class is whether it is the boundary point of the $\beta$ or $\gamma$ region.

**Quantitative calculation**

$$V = (S_{ELP} - S_x) * K$$

V: Quantitative results
$S_{ELP}$: the area of ELP curve in $\beta$ region or $\gamma$ region
$S_x$: the area of M protein curve in $\beta$ region or $\gamma$ region
K: coefficient

K is used for dimension conversion, i.e., unit conversion.
Through the above method, as shown in FIG. 5, the quantitative result of the M protein is calculated as 3.89 g/l.
The above descriptions are only specific preferred implementations of the present application, but the scope of protection of the present application is not limited thereto. Any technical personnel familiar with the technical field who, within the scope of the technology disclosed in this application, make equivalent substitutions or changes based on the technical solution and invention concept of this application shall be covered within the scope of protection of this application.

**Industrial applicability**

The present application provides a quantitative analysis method of a capillary immunotyping monoclonal immune globulin. In order to solve the problem of M protein type identification, the present application combines an immunochemical method and electrophoretic method by adopting a capillary immunotyping technology, wherein electrophoresis is performed after a specific antibody and a corresponding M protein form an immunocomplex, thus the M protein peak will be eliminated, and the M protein can be accurately quantified by a difference value between peak areas of two electrophoresis. This method of present application can objectively and accurately distinguish the M protein from other non-immune globulins, thereby avoiding the subjectivity and poor precision of manual quantification.

**Claims**

1. A quantitative analysis method of a capillary immunotyping monoclonal immune globulin, wherein a region boundary of a compound curve is determined through curve derivation or a neural network, and then quantitative calculation is performed to obtain a quantitative result of an M protein;
   the compound curve is obtained by the following method:

   performing electrophoresis on a monoclonal immune globulin serum sample to obtain an M protein curve;
   mixing the monoclonal immune globulin serum sample with an antibody to form an immunocomplex and then performing electrophoresis again to obtain an M protein post-reaction curve; and
   compounding the M protein curve with the M protein post-reaction curve to obtain the compound curve.

2. The quantitative analysis method of a capillary immunotyping monoclonal immune globulin according to claim 1, comprising the following steps:

   S1 obtaining a M protein curve and an M protein post-reaction curve: performing electrophoresis on the monoclonal immune globulin serum sample to obtain the M protein curve, mixing the monoclonal immune globulin serum sample with the antibody to form the immunocomplex and then performing electrophoresis again to obtain the M protein post-reaction curve;
   S2 compounding and determining a region boundary line: compounding the M protein curve with the M protein post-reaction curve to obtain the compound curve, and determining the region boundary of the compound curve through curve derivation or the neural network;
   the region boundary comprises a left boundary line of a β region, a right boundary line of the β region, a left boundary line of a γ region and a right boundary line of the γ region;
   a method for determining the region boundary through curve derivation is as follows: calculating a first-order derivative and a second-order derivative of each point on the M protein curve, and determining the region boundary according to the values of the first-order derivative and the second-order derivative;
   S3 quantitative calculation: calculating the area $S_{ELP}$ of the M protein curve in the β region or the γ region according to the left boundary line of the β region, the right boundary line of the β region or the left boundary line of the γ region, the right boundary line of the γ region, calculating the area $S_x$ of the M protein post-reaction curve in the β region or the γ region according to the left boundary line of the β region, the right boundary line of the β region and/or the left boundary line of the γ region, the right boundary line of the γ region, and calculating the quantitative result V of the M protein according to the following equation:

$$V = (S_{ELP} - S_x)*K,$$

   wherein, K is a coefficient.

3. The quantitative analysis method of a capillary immunotyping monoclonal immune globulin according to claim 1, wherein the antibody comprises IgA antibody, IgG antibody and IgM antibody, each of which comprises K type and L type;
   both the M protein curve and the M protein post-reaction curve comprise 300 coordinate points.

4. The quantitative analysis method of a capillary immunotyping monoclonal immune globulin according to claim 2, wherein in step S2, a coordinate point position where the first-order derivative is 0 is a wave peak or wave trough of the M protein curve, the left boundary line of the β region is a first wave trough in a designated region, the right boundary line of the β region is a second wave trough or a position where a first second-order derivative is 0 after a first peak, the left boundary line of the γ region is a first wave trough after the right boundary line of the β region, the right boundary line of the γ region is a first coordinate point position where the difference between the M protein curve and the M protein post-reaction curve is 0.

5. The quantitative analysis method of a capillary immunotyping monoclonal immune globulin according to claim 2, wherein,
   in step S2, the equation of the first-order derivative is:

$$f'(x_0) = \lim_{\Delta x \to 0} \frac{\Delta y}{\Delta x} = \lim_{\Delta x \to 0} \frac{f(x_0 + \Delta x) - f(x_0)}{\Delta x}$$

6. The quantitative analysis method of a capillary immunotyping monoclonal immune globulin according to claim 2, wherein,
in step S2, the equation of the second-order derivative is:

$$f''(x) = \lim_{\Delta x \to 0} \frac{f'(x_0 + \Delta x) - f'(x_0)}{\Delta x}$$

.

7. The quantitative analysis method of a capillary immunotyping monoclonal immune globulin according to claim 2, wherein in step S2, a method for determining the region boundary through neural network is as follows: performing scaling and residual block convolution operation on the image of the compound curve to obtain a feature map and ultimately obtain a feature vector, performing classification by utilizing the feature vector to finally obtain n classification results, and then determining whether each of the classification results is the left boundary line of the $\beta$ region, the right boundary line of the $\beta$ region, the left boundary line of the $\gamma$ region or the right boundary line of the $\gamma$ region, then obtaining the region boundary.

8. The quantitative analysis method of a capillary immunotyping monoclonal immune globulin according to claim 7, wherein in step S2, a method for obtaining the classification results is as follows: resizing the image of the compound curve to obtain a 224*224 image which is fed into a first residual block, performing four convolution operations to obtain a 220*220 feature map, interpolating the 224*224 image to obtain a 220*220 image, adding the 220*220 image and the 220*220 feature map to obtain a 220*220 new feature map;

then, down-sampling twice the 220*220 new feature map to obtain a 110*110 feature map which is fed into a second residual block, performing four convolution operations to obtain a 106*106 new feature map, interpolating the 110*110 feature map to obtain a 106*106 feature map, adding the 106*106 feature map and the 106*106 new feature map to obtain a 106*106 fused feature map, and down-sampling twice the 106*106 fused feature map to obtain a 53*53 feature map;
the above operations are repeated to finally obtain a 10*10 feature map, which is global average pooled to obtain a 2560-dimensional feature vector, and classification is performed by utilizing the 2560-dimensional feature vector to finally obtain n of the classification results.

9. The quantitative analysis method of a capillary immunotyping monoclonal immune globulin according to claim 8, wherein n is 300.

10. The quantitative analysis method of a capillary immunotyping monoclonal immune globulin according to claim 2, wherein in step S2, a method for obtaining the compound curve is as follows: inputting the M protein curve and the M protein post-reaction curve into a database respectively, then parsing all the data of the database to call out the M protein curve, calling out the M protein post-reaction curve from the database by being paired with the M protein curve, and then compounding the M protein curve with the M protein post-reaction curve to obtain the compound curve.

Performing electrophoresis on a monoclonal immune globulin serum sample to obtain an M protein curve, mixing the monoclonal immune globulin serum sample with an antibody to form an immunocomplex and then performing electrophoresis again to obtain an M protein post-reaction curve `S1`

Compounding the M protein curve with the M protein post-reaction curve to obtain the compound curve, and then determining the region boundary of the compound curve through curve derivation or a neural network `S2`

Calculating the area of the M protein curve in the β region or the γ region according to the region boundary on the M protein curve, calculating the area of the M protein post-reaction curve in the β region or the γ region according to the region boundary, and calculating the quantitative result of the M protein `S3`

FIG. 1

FIG. 2

FIG. 3

FIG. 4

FIG. 5

## INTERNATIONAL SEARCH REPORT

| | International application No. |
|---|---|
| | **PCT/CN2022/086799** |

**A. CLASSIFICATION OF SUBJECT MATTER**

G01N 33/68(2006.01)i;  G01N 27/447(2006.01)i;  G06N 3/02(2006.01)i

According to International Patent Classification (IPC) or to both national classification and IPC

**B. FIELDS SEARCHED**

Minimum documentation searched (classification system followed by classification symbols)

G01N33 G01N27 G06N3

Documentation searched other than minimum documentation to the extent that such documents are included in the fields searched

Electronic data base consulted during the international search (name of data base and, where practicable, search terms used)

CNABS; CNTXT; CNKI; VEN; USTXT; WOTXT; EPTXT; ISI Web of Science: 毛细管电泳, 电泳, 单克隆免疫球蛋白, M蛋白, 免疫, 分型, 免疫消减, 导数, 微分, 神经网络, capillary electrophoresis, monoclonal protein, M-protein, immunosubtraction, derivative, neural network

**C. DOCUMENTS CONSIDERED TO BE RELEVANT**

| Category* | Citation of document, with indication, where appropriate, of the relevant passages | Relevant to claim No. |
|---|---|---|
| PX | CN 113552370 A (BEIJING XIAOYING TECHNOLOGY CO., LTD.) 26 October 2021 (2021-10-26)<br>claims 1-10 | 1-10 |
| Y | US 5567282 A (WANG HANN-PING et al.) 22 October 1996 (1996-10-22)<br>abstract, column 5, line 47 to column 6, line 34, column 7, lines 31-37, column 8, lines 29-46, and figure 1 | 1-10 |
| Y | MAGNUS JONSSON et al. "Computer-supported Detection of M-Components and Evaluation of Immunoglobulins after Capillary Electrophoresis"<br>*CLINICAL CHEMISTRY*, Vol. 47, No. 1, 31 January 2001 (2001-01-31),<br>ISSN: 0009-9147,<br>page 111, right-hand column, paragraph 4 | 1-6, 10 |
| Y | CN 110009008 A (SICHUAN UNIVERSITY) 12 July 2019 (2019-07-12)<br>description, paragraphs [0043] and [0080]-[0083] | 1-3, 7-10 |
| A | CN 110857929 A (ARKRAY INC.) 03 March 2020 (2020-03-03)<br>entire document | 1-10 |

☑ Further documents are listed in the continuation of Box C.   ☑ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| **24 May 2022** | **27 June 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/086799** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP H05312812 A (JOKO KK) 26 November 1993 (1993-11-26)<br>    entire document | 1-10 |
| A | US 6156179 A (BIO-RAD LABORATORIES, INC.) 05 December 2000 (2000-12-05)<br>    entire document | 1-10 |

Form PCT/ISA/210 (second sheet) (January 2015)

**INTERNATIONAL SEARCH REPORT**
Information on patent family members

| | | | International application No. |
|---|---|---|---|
| | | | **PCT/CN2022/086799** |

| Patent document cited in search report | | | Publication date (day/month/year) | Patent family member(s) | | | Publication date (day/month/year) |
|---|---|---|---|---|---|---|---|
| CN | 113552370 | A | 26 October 2021 | CN | 113552370 | B | 28 December 2021 |
| US | 5567282 | A | 22 October 1996 | WO | 9520160 | A2 | 27 July 1995 |
| | | | | EP | 0690988 | A1 | 10 January 1996 |
| | | | | ES | 2144604 | T3 | 16 June 2000 |
| | | | | JP | H08508576 | A | 10 September 1996 |
| | | | | DE | 69516048 | D1 | 11 May 2000 |
| | | | | WO | 9520160 | A3 | 24 August 1995 |
| | | | | EP | 0690988 | B1 | 05 April 2000 |
| CN | 110009008 | A | 12 July 2019 | CN | 110009008 | B | 06 November 2020 |
| CN | 110857929 | A | 03 March 2020 | EP | 3614134 | A1 | 26 February 2020 |
| | | | | US | 2020072790 | A1 | 05 March 2020 |
| | | | | JP | 2020030116 | A | 27 February 2020 |
| | | | | EP | 3614134 | B1 | 03 March 2021 |
| JP | H05312812 | A | 26 November 1993 | JP | 3390873 | B2 | 31 March 2003 |
| US | 6156179 | A | 05 December 2000 | AU | 3913799 | A | 10 February 2000 |
| | | | | EP | 0990901 | A2 | 05 April 2000 |
| | | | | CA | 2275853 | A1 | 09 January 2000 |
| | | | | CA | 2275853 | C | 11 June 2002 |
| | | | | EP | 0990901 | A3 | 15 May 2002 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111114069